(19) Europäisches Patentamt
European Patent Office
Office européen des brevets

(11) Publication number: **0 129 343**
**A2**

# (12) EUROPEAN PATENT APPLICATION

(21) Application number: 84303423.2

(22) Date of filing: 21.05.84

(51) Int. Cl.³: C 08 G 63/08
C 07 D 307/62

(30) Priority: 19.05.83 US 496287

(43) Date of publication of application:
27.12.84 Bulletin 84/52

(84) Designated Contracting States:
DE FR GB IT

(71) Applicant: Markham, Richard Glover
945 Country Club Drive
Prescott Arizona 86301(US)

(71) Applicant: Elders, Gerald Wayne
38 Yakashba Estates
Prescott Arizona 83601(US)

(71) Applicant: Webb, Rockwell Colton
128 S. Mount Vernon Street
Prescott Arizona 86301(US)

(72) Inventor: Markham, Richard Glover
945 Country Club Drive
Prescott Arizona 86301(US)

(74) Representative: Stephens, Michael John
M.J. Stephens & Co. 46 Tavistock Place
Plymouth PL4 8AX(GB)

## (54) Ascorbic acid derivatives and a method for their preparation.

(57) Polycondensed ester derivatives of ascorbic acid are described and prepared by the polycondensation of ascorbic acids and/or its derivative dehydroascorbic acid in a sealed container in the presence of water to form the respective self esters or self-ester and co-ester mixtures. Alternatively ascorbic acid is partly converted to its calcium salt and polycondensed in the absence of oxygen to form its self-ester or in the presence of oxygen to form dehydroascrobic acid selfesters or mixtures of self-esters and co-esters of the two acids.

EP 0 129 343 A2

Croydon Printing Company Ltd.

# ASCORBIC ACID DERIVATIVES AND A METHOD FOR THEIR PREPARATION

This invention relates to novel derivatives of L-ascorbic acid and its derivative, dehydroascorbic acid,and to a method for their preparation.

The invention provides novel derivatives of ascorbic acid having the structure

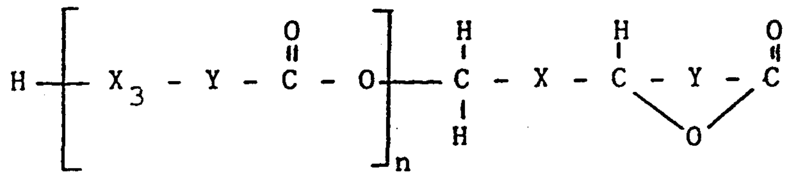

in which n is a positive integer; X is the group 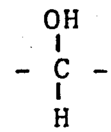

and Y is the group: 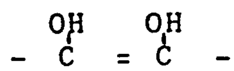 or

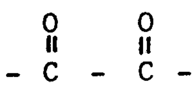 , the Y groups being the same or different, possibly in the form of a metal salt.

Another aspect of the invention provides a method for the preparation of the above derivatives comprising heating L-ascorbic acid and/or dehydroascorbic acid with water to effect polycondensation thereof: the derivatives in which each Y group is 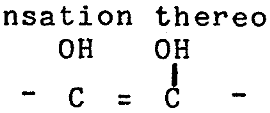 are formed by the self-esterification of L-ascorbic acid, according to the equation;

n + 1 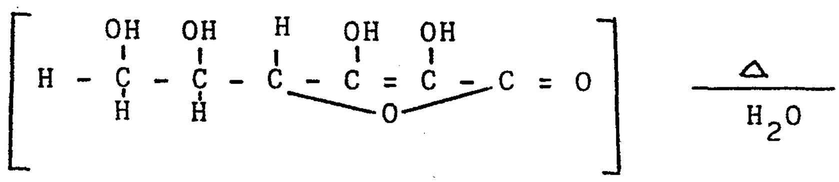


(L-ascorbic acid)

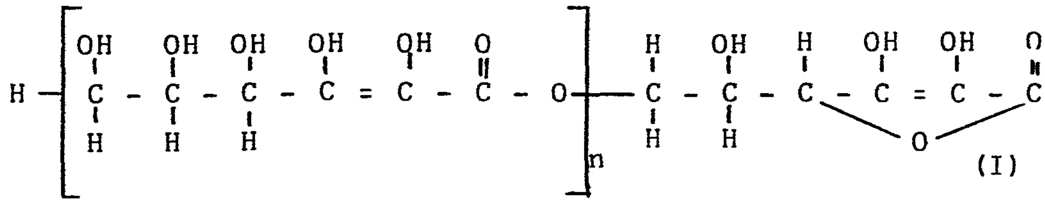

(I)

The derivatives in which each Y group is 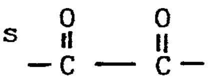

are formed by the self-esterification of dehydroascorbic acid according to the equation;

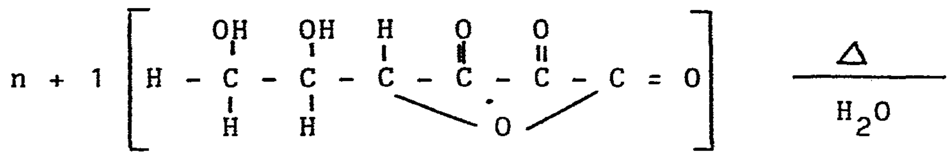


(dehydroascorbic acid)

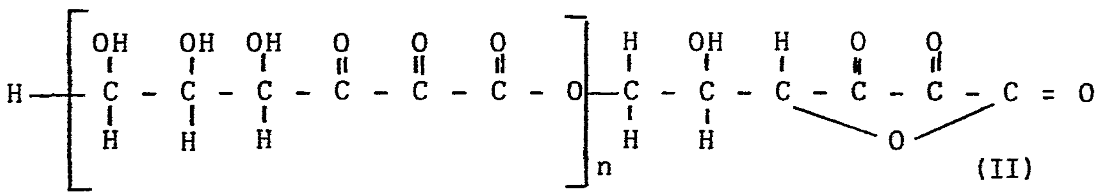

(II)

Derivatives in which the Y groups differ are formed by the polycondensation of ascorbic acid and dehydroascorbic acid mixtures.

The polycondensation reactions may be carried out by heating the ascorbic or dehydroascorbic acid or mixtures thereof carefully in the presence of water in sealed containers, such as glass tubes, at a temperature of the order of 100°C until the esterification reaction is complete. Water in the esterification mixture shifts the equilibrium between the open-chain and cyclic lactone forms of ascorbic acid towards the cyclic form:e.g.

Equilibrium of straight chain and cyclic lactone forms of ascorbic acid

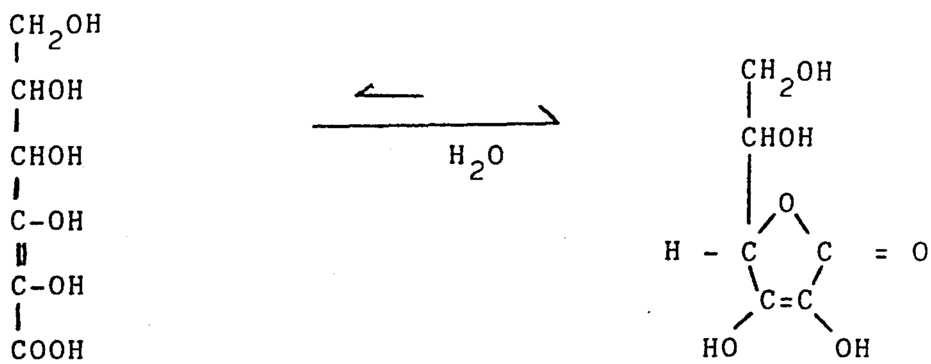


This, in turn, favours the esterification reactions.

It is found, in practice, that the reaction continues as long as there is unreacted acid present, yielding products of increasingly higher molecular weights as the reaction continues. Broad infrared absorbance peaks in the region 1600-1700 $cm^{-1}$ , characteristic of the cyclic rings of the acids, diminish as the reactions proceed and are at very low levels at molecular weights of the reaction products corresponding to the tetramer.

Ultimate analyses of products eluted from reaction mixtures by gel chromatography at MW 700 and higher confirm the predicted empirical formulae for the products (I) and (II) at $n \geq 3$. Product (I) is colourless and product (II) is yellow.

Products (I) and (II) and mixtures thereof are useful in treatment of ideopathic movement disorders in laboratory animals and are confirmed non-toxic by rat-feeding studies.

An alternative preparation of the compounds of the invention involves the partial conversion of an aqueous L-ascorbic acid solution to a metal salt, advantageously the calcium salt, to provide a non-toxic product, and the heating of the aqueous acid-salt mixture at a temperature in the range 80-95°C for a length of time sufficient to attain the desired degree of esterification. The conversion of unconverted acid in the esterification mixture is then preferably completed by reaction with excess salt-conversion reagent e.g., calcium carbonate, and the reaction mixture is dried. The esterified product including metal salts thereof, may be separated from the unreacted reagent, e.g., by water-leaching the soluble product out of the dried reaction mixture, and the leach solution may then be dried to give a product containing the ester derivatives and salts thereof, which may be used as such or subjected to gel separation to isolate the higher molecular weight esters and salts thereof from the lower molecular weight species and the salts.

If solely the ascorbic acid self-ester product is required, the preparation is carried out in the absence of oxygen. If, on the other hand, the dehydroascorbate self-ester product is desired, all or a portion of the L-ascorbic acid can be oxidised by bubbling air through the reaction mixture after the partial salt conversion step described above and before the esterification, drying and separation steps.

Several methods of preparation of the derivatives of the invention will now be more particularly described, by way of example.

Example 1

L-ascorbic acid is moistened with distilled water and placed in sealed and open glass tubes in an oven preheated to 100°C. After six hours the tubes are withdrawn and examined by infrared spectroscopy. The materials in the sealed tubes do not show infrared absorption bands in the 1600-1700 $cm^{-1}$ band while those in the open tubes clearly show such bands characteristic of the lactone ring structure of L-ascorbic acid. The dry residue in the open tubes is dark brown. The ascorbic acid self-ester product, isolated from the material in the sealed tubes by gel chromatography using Sephadex G-10, contains molecular weight species varying from 425 to 700.

Example 2

To a 2-litre , 3-neck flask equipped with an agitator, thermometer and vent tube is added 300 ml distilled water and 440 g (2.5 moles) L-ascorbic acid. To this stirred slurry, finely divided calcium carbonate is added incrementally at a rate such as to produce a constant evolution of carbon dioxide(reaction byproduct)the reaction temperatûre being maintained at about 20°C. The addition of calcium carbonate is suspended after about 25 g to 37.5 g have been added (representing from about 20% to 30% of that required for complete reaction with the L-ascorbic acid charge).

At this point the temperature is raised to 80°C while an atomosphere of carbon dioxide is maintained in the reactor. At intervals, samples of the reaction mixture are removed and analysed by infrared spectrometry to determine the degree of L-ascorbic acid esterification which has taken place , further additions of calcium

carbonate are begun, the temperature being maintained in the range 60°C to about 70°C, and an oxygen-free atmosphere still being maintained in the reactor. The total quantity of calcium carbonate added is 125 g (1.25 moles).

The reaction mixture is transferred to a covered, shallow container in an essentially oxygen-free atmosphere, maintained at a temperature of between 60°C and 80°C, for a period of from 12 to 36 hours, during which time the pH of the mixture rises to a pH range of 6.0-7.0. At this point, the excess water is removed under vacuum.

The dry products are light tan in colour and readily soluble in water, except for unreacted calcium carbonate, to produce neutral solutions. Analysis indicates that the products possess molecular weights averaging about 460. By gel filtration techniques using Sephadex G-10, a product is eluted at the solvent front which, when isolated, has molecular weights in excess of 700. This corresponds to self-ester species having at least four ascorbic acid units. Some units contain calcium.

## Example 3

The procedures of Example 2 are followed, with the exception that air is not excluded from the reaction mixture throughout the operation. This leads to the intermediate formation of dehydroascorbic acid and resultant self-esterified dehydroascorbic acid and salts thereof.

The quantities of the oxidation products are regulated by the extent of air contact within the reactor and during the subsequent heating and drying periods.

The dry products generally have similar physical characteristics to those described in Example 1. They are usually darker in colour, being light brown.

CLAIMS

1. A derivative of ascorbic acid having the structure:

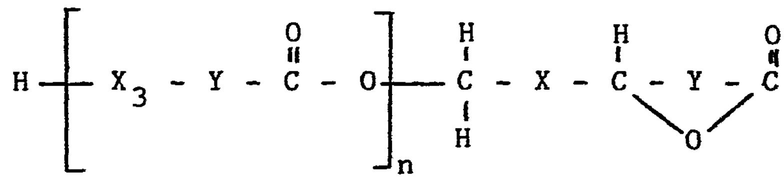

in which n is a positive integer; X is the group 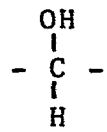

and Y is the group: 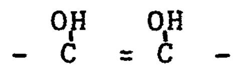 or

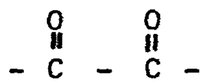, the Y groups being the same or different.

2. A derivative according to Claim 1, characterised in that it is in the form of a metal salt.

3. A method of preparing an ascorbic acid derivative according to Claim 1 or Claim 2, comprising heating ascorbic acid and/or dehydroascorbic acid with water to effect polycondensation thereof, the polycondensation of ascorbic acid providing derivatives in which each Y group is the group 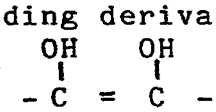, the polycondensation of dehydroascrobic acid providing derivations in which each Y group is 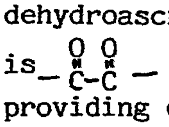, and the polycondensation of a mixture of the acids providing derivatives with mixed Y groups.

4. A method according to Claim 3, characterised in that the acid or mixture thereof is heated in a sealed container, in the presence of water, to a temperature of the order of 100°C.

5. A method according to Claim 3, characterised in that ascorbic acid is at least partially converted to a metal salt and the reaction mixture, including unreacted acid and the salt, is heated to a temperature of substantially 80°C to 95°C to effect the polycondensation.

6. A method according to Claim 5,characterised in that the heating is carried out in the absence of oxygen to form the self-esterified derivative of ascorbic acid.

7. A method according to Claim 4, characterised in that the heating is carried out in the presence of oxygen so as to convert some of the ascorbic acid to dehydroascorbic acid prior to the polycondensation reaction.

8. A method according to any one of Claims 5 to 7, characterised in that calcium carbonate is added to the ascorbic acid in water to convert part of the acid to the calcium salt and the temperature of the mixture is then raised to effect the polycondensation.

9. A method according to Claim 8, characterised in that the temperature is lowered when a desired degree of esterification has taken place and further calcium carbonate is added to convert unreacted acid to the calcium salt.